# EUROPÄISCHE PATENTSCHRIFT

(11) **EP 3 551 267 B1**
(45) Veröffentlichungstag und Bekanntmachung des Hinweises auf die Patenterteilung: **11.03.2020**
(21) Anmeldenummer: 18826324.8
(22) Anmeldetag: 19.12.2018
(51) Int. Cl.: A61F 2/20, A61M 16/04, A61M 16/10, A61M 16/20

(54) **WÄRME- UND FEUCHTIGKEITSAUSTAUSCHER**
HEAT AND MOISTURE EXCHANGER
ÉCHANGEUR DE CHALEUR ET D'HUMIDITÉ

(30) Priorität: 20.12.2017 DE 102017130662
(43) Veröffentlichungstag der Anmeldung: 16.10.2019
(73) Patentinhaber: Andreas Fahl Medizintechnik-Vertrieb GmbH, 51149 Köln (DE)
(72) Erfinder: FAHL, Andreas, 51107 Köln (DE)
(74) Vertreter: Geskes, Christoph
(86) Internationale Anmeldenummer: PCT/EP2018/085814
(87) Internationale Veröffentlichungsnummer: WO 2019/121894

(56) Entgegenhaltungen:
- EP-A1- 2 236 165
- WO-A1-2006/118599
- DE-A1-102012 024 817
- DE-B3-102014 002 064

## Beschreibung

Die Erfindung betrifft einen Wärme- und Feuchtigkeitsaustauscher für Tracheostomierte oder Laryngektomierte, der eine Ventilplatte und ein Gehäuse umfasst.

Wärme- und Feuchtigkeitsaustauscher für Tracheostomierte oder Laryngektomierte sind aus dem allgemeinen Stand der Technik bereits bekannt. Die WO 2010/060983 A1 beschreibt einen sogenannten Atemschutz, der ein Gehäuse und eine Ventilplatte aufweist. Im Gehäuse befindet sich ein Filter, der sich nach distal bis zu der Ventilplatte erstreckt. Durch Druck auf die Ventilplatte wird der Filter zusammengepresst und die Ventilplatte auf einen Ventilsitz gedrückt, der Teil des Gehäuses ist, und somit wird der Atemschutz verschlossen. Dies hat den Nachteil, dass beim Verschließen des Wärme- und Feuchtigkeitsaustauschers die im Filtermaterial befindliche Flüssigkeit ausgedrückt wird und in die Trachea des Benutzers oder auf dessen Kleidung fließt. Die Ventilplatte wird des Weiteren mittels einer an eine Filteraufnahme angrenzenden käfigartigen Führung transversal geführt. Die Ventilplatte kann, je nach Einleitungsstelle der Kraft in die Ventilplatte, dabei gegebenenfalls leicht kippen und verklemmen. Ein weiter Nachteil ist eine eher ungenaue taktile Rückmeldung beim Betätigen des Wärme- und Feuchtigkeitsaustauschers, die der Benutzer unangenehm empfinden kann. Die dort offenbarte Ventilplatte weist des Weiteren Vorsprünge auf, die radial nach außen vorstehen und in Öffnungen eingreifen, damit die Ventilplatte in ihrer Bewegung nach distal begrenzt wird und nicht verloren geht. Ein solcher Wärme- und Feuchtigkeitsaustauscher hat den Nachteil, dass seine Montage etwas aufwändig ist.

Nachfolgende Dokumente offenbaren auch einen Wärme- und Feuchtigkeitsaustauscher DE 10 2012 024817, EP 2 236 165, WO 2006/118599, DE 10 2014 002064.

Insbesondere ist Aufgabe der vorliegenden Erfindung, eine Benutzerfreundlichkeit eines Wärme- und Feuchtigkeitsaustauschers zu verbessern. Weiterhin ist es Aufgabe der Erfindung, eine Dichtheit des Wärme- und Feuchtigkeitsaustauschers zu verbessern.

Diese Aufgabe wird erfindungsgemäß in den angehängten Ansprüchen gelöst mittels eines Wärme- und Feuchtigkeitsaustauschers für Tracheostomierte oder Laryngektomierte, zumindest umfassend eine Ventilplatte und ein Gehäuse, wobei die Ventilplatte eine radial umlaufende Lippe umfasst und wobei das Gehäuse einen eine distale Öffnung des Gehäuses umschließenden Ventilsitz umfasst, wobei der Ventilsitz eine Nut zur Aufnahme der Lippe umfasst, wobei die Ventilplatte der distalen Öffnung des Gehäuses zugeordnet ist und wobei die Ventilplatte in eine Geschlossenstellung überführbar ist, bei der die Lippe in die Nut eingreift. Weiterhin wird die Aufgabe erfindungsgemäß gelöst mittels eines Verfahrens zum Verschließen eines Wärme- und Feuchtigkeitsaustauschers, wobei eine Ventilplatte nach proximal bewegt wird, wobei eine Lippe der Ventilplatte in eine Nut eines Gehäuses eingreift. Der erfindungsgemäße Wärme- und Feuchtigkeitsaustauschers wird zur Umleitung eines Luftstromes verwendet.

Es wird ein Wärme- und Feuchtigkeitsaustauscher für Tracheostomierte oder Laryngektomierte vorgeschlagen. Dieser umfasst eine Ventilplatte und ein Gehäuse. Die Ventilplatte umfasst eine radial umlaufende Lippe. Das Gehäuse umfasst einen eine distale Öffnung des Gehäuses umschließenden Ventilsitz, wobei der Ventilsitz eine Nut zur Aufnahme der Lippe umfasst. Die Ventilplatte ist der distalen Öffnung des Gehäuses zugeordnet, wobei die Ventilplatte in eine Geschlossenstellung überführbar ist, bei der die Lippe in die Nut eingreift.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeatmet werden kann. Beispielsweise bei laryngektomierten, also kehlkopflosen Personen, bei welchen der Kehlkopf operativ entfernt wurde, muss das Tracheostoma dauerhaft offen gehalten und stabilisiert werden, wozu insbesondere Trachealkanülen, in der Regel aus einer Außen- und einer Innenkanüle bestehend, in das Tracheostoma eingesetzt werden. Aber auch der Einsatz so genannter "Tracheostoma-Button" ist möglich, insbesondere für Personen, die keine Trachealkanüle mehr benötigen.

Des Weiteren können in das Tracheostoma auch so genannte "Shunt-Ventile" eingesetzt werden, welche eine Wiederherstellung der Stimme ermöglichen. Schließlich können in das Tracheostoma auch Filtersysteme sowohl bei Tracheotomierten als auch bei Laryngektomierten eingesetzt werden. Derartige Filtersysteme bestehen insbesondere aus einem Pflaster mit einem eingesetzten Filter in einem Gehäuse und/oder Filtermaterial oder aus einer üblicherweise selbstklebenden Basisplatte - in aller Regel aus Kunststoff gebildet-, in welche Filter in einem Gehäuse und/oder Filtermaterial unterschiedlichster Art einsetzbar sind.

Zu den Filtersystemen, welche für laryngo-tracheale Hilfsmittel wie Trachealkanülen, Shunt-Ventile, Tracheostoma-Button und Filtersysteme mit Pflaster oder Basisplatte eingesetzt werden, zählen die gattungsgemäßen Wärme- und Feuchtigkeitsaustauscher, auch "künstliche Nase" genannt. Diese dienen dazu, die bei laryngektomierten und auch tracheotomierten Menschen fehlenden Regulationsmechanismen zur Erwärmung und Befeuchtung der Atemluft nachzubilden und ein In-Kontakt-Bringen der Luftröhre mit verstärkt trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Wärme- und Feuchtigkeitsaustauscher wird die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Gattungsgemäße Wärme- und Feuchtigkeitsaustauscher für Laryngektomierte und auch Tracheotomierte weisen ein Filtermaterial auf - in aller Regel aus Papier oder Schaumstoff gebildet - durch welches die ein- und ausgeatmete Luft geleitet wird. Beim Ausatmen hält das Filtermaterial Feuchtigkeit zurück, welche dann beim Einatmen in die Luftröhre transportiert wird. Aus dem Stand der Technik bekannte Wärme- und Feuchtigkeitsaustauscher gibt es in einer großen Vielzahl unterschiedlicher Ausbildungen für unterschiedliche Adapter. Der Universaladapter gemäß DIN EN ISO 5356-1 weist einen Durchmesser von 15 mm auf, es sind jedoch aus dem Stand der Technik auch weitere Adapter mit 22 mm Durchmesser bekannt.

Ein weiteres Hilfsmittel für Laryngektomierte sind Stimmprothesen, die der Wiederherstellung der Stimme nach einer Laryngektomie dienen. Die Wiederherstellung der Stimme kann durch chirurgische Maßnahmen erfolgen. Wichtigstes Hilfsmittel dabei ist die Stimmprothese, die auch als "Shunt-Ventil" bezeichnet wird. Mittels einer Stimmprothese können Funktionen des entfernten Kehlkopfes ersetzt werden. Zum einen wird durch den Einsatz einer Stimmprothese eine Luftzufuhr von der Lunge über die Speiseröhre (Ösophagus) in den Rachen ermöglicht. Die Ausatemluft wird dabei zum Sprechen verwendet. Zum anderen dichtet die Stimmprothese die Verbindung von der Speiseröhre zur Luftröhre (Trachea) beim Schlucken von Speisen und Getränken ab, wodurch der Nutzer der Stimmprothese vor einem versehentlichen Verschlucken der Nahrung geschützt ist.

Sind die Wunden nach dem operativen Eingriff geheilt, können die Menschen im Normalzustand nicht sprechen. Um ein Sprechen zu ermöglichen, ist bei Verwendung einer Stimmprothese oder der noch vorhandenen Larynx bei tracheotomierten Menschen der Luftaustritt am Hals zu verschließen, damit die Luft durch die Stimmprothese beziehungsweise die Larynx geleitet wird. Hierzu weist der erfindungsgemäße Wärme- und Feuchtigkeitsaustauscher die Ventilplatte zur Bildung einer Ventilfunktion in diesem auf.

In einer Ausgestaltung ist vorgesehen, dass die Ventilplatte als Deckelteil ausgestaltet ist. Besonders bevorzugt ist die Ventilplatte kreisrund ausgestaltet. In einer weiteren Ausgestaltung ist vorgesehen, dass die Ventilplatte plattenförmig ausgestaltet ist. In einer weiteren Ausführungsform weist die Ventilplatte in einem Querschnitt eine zumindest teilweise geschwungene Formgebung auf. Insbesondere ist die zumindest teilweise geschwungene Formgebung auf einer distalen Seite der Ventilplatte angeordnet. Weiter bevorzugt ist die proximale Seite der Ventilplatte im Wesentlichen eben ausgestaltet, mit Ausnahme der Lippe. Insbesondere weist die Ventilplatte in zumindest einem Querschnitt eine geschwungene Formgebung der Oberfläche, insbesondere der distalen Seite der Oberfläche, auf. In einer weiteren Ausführungsform ist vorgesehen, dass die Ventilplatte, insbesondere die distale Oberfläche der Ventilplatte, insbesondere im Wesentlichen zentral eine Vertiefung aufweist.

In einer weiteren Ausführungsform ist vorgesehen, dass die Ventilplatte insbesondere auf der distalen Seite eine, insbesondere konzentrisch, umlaufenden Wölbung aufweist. Bevorzugt ist die Wölbung ringförmig ausgestaltet. Eine konzentrisch angeordnete Wölbung auf der distalen Oberfläche der Ventilplatte weist in einer Ausgestaltung in einem Schnitt von distal nach proximal durch diese einen Wölbungsradius von etwa 10 mm bis etwa 20 mm, weiter bevorzugt etwa 12 mm bis etwa 16 mm, weiter bevorzugt etwa 13 mm bis etwa 15 mm, weiter bevorzugt etwa 14 mm, weiter bevorzugt etwa 14,14 mm auf. In einer weiteren Ausführungsform ist vorgesehen, dass eine Vertiefung in einem Schnitt von distal nach proximal einen Radius von etwa 30 mm bis etwa 50 mm, weiter bevorzugt etwa 35 mm bis etwa 45 mm, weiter bevorzugt etwa 40 mm aufweist. Bevorzugt ist die Wölbung um die Vertiefung herum angeordnet. In einer weiteren Ausführungsform ist vorgesehen, dass die proximale, der Wölbung und Vertiefung gegenüberliegende Seite im Wesentlichen eben ausgestaltet ist. Bevorzugt ist die Vertiefung im Inneren der ringförmigen Wölbung kreisscheibenartig ausgebildet.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen.

In einer weiteren bevorzugten Ausgestaltung ist vorgesehen, dass die Ventilplatte luftdicht ausgestaltet ist. Vorzugsweise umfasst die Ventilplatte keinerlei Öffnungen, die einen Durchtritt von Gas beziehungsweise Luft durch die Ventilplatte erlauben. In einer weiteren Ausführungsform ist vorgesehen, dass die Ventilplatte eine radial umlaufende Lippe aufweist, die bevorzugt nach proximal ragt. Die Lippe ragt in einer Ausführungsform ausgehend von einer proximalen Oberfläche der Ventilplatte etwa 0,5 mm bis etwa 1,5 mm, weiter bevorzugt etwa 0,5 mm bis etwa 1 mm, weiter bevorzugt etwa 0,7 mm, nach proximal. Weiterhin bevorzugt ist vorgesehen, dass die Lippe einem Rand der Ventilplatte zugeordnet ist. In einer weiteren Ausführungsform ist vorgesehen, dass die Ventilplatte materialverbunden ist. In einer weiteren Ausführungsform ist vorgesehen, dass die Lippe an die Ventilplatte angespritzt ist. In einer Ausgestaltung umfasst die Lippe ein gummielastisches Material, wie beispielsweise insbesondere medizinisches Silikon oder Kautschuk. Sie kann derart bevorzugt im 2-Komponenten-Spritzgussverfahren mit der eigentlichen Ventilplatte, die aus einem härteren Material als die Lippe ist, hergestellt werden. In einer weiteren Ausgestaltung umfasst die Lippe ein starres Material, beispielsweise ein Material ausgewählt aus einer Gruppe umfassend Polyamid, Polycarbonat, Acrylnitril-Butadien-Styrol-Copolymere (ABS) und/oder eine Mischung aus zumindest zwei der genannten Materialien. Weiterhin ist in einer Ausführungsform vorgesehen, dass die Lippe das gleiche Material umfasst wie die Ventilplatte.

Die Lippe ist insbesondere eine Materialerstreckung, die von einer im Wesentlichen ebenen Oberfläche der Ventilplatte bevorzugt in Richtung proximal ragt. Vorzugsweise ist die Lippe radial umlaufend, insbesondere vollständig umlaufend ausgebildet. Weiter bevorzugt ist die Lippe einer Außenkante der Ventilplatte zugeordnet, insbesondere bildet die Lippe die Außenkante der Ventilplatte. In einer weiteren Ausgestaltung ist die Lippe in einem Querschnitt im Wesentlichen rechteckig ausgestaltet. In einer weiteren Ausgestaltung ist die Lippe im Wesentlichen trapezförmig ausgestaltet.

Die im Rahmen der Erfindung verwendeten Richtungsangaben sind gemäß der Erfindung in Bezug auf den bestimmungsgemäßen Einbau an einer Trachealkanüle oder einem Tracheostomapflaster beziehungsweise am Körper einer Benutzers zu verstehen. Unter dem Begriff "distal" wird im Sinne der vorliegenden Erfindung in Bezug auf ein Merkmal der erfindungsgemäßen Vorrichtung eine Anordnung beziehungsweise Verwendung desselben fern oder auch abgewandt oder gegenüberliegend einem Tracheostomapflaster oder einer Trachealkanüle, allgemeiner einer Hautoberfläche eines Benutzers, der insbesondere solche Befestigungsmittel für die erfindungsgemäße Vorrichtung trägt, verstanden. Unter dem Begriff "proximal" wird im Sinne der vorliegenden Erfindung in Bezug auf ein Merkmal der erfindungsgemäßen Vorrichtung eine Anordnung beziehungsweise Verwendung desselben nah oder auch zugewandt oder benachbart einem Tracheostomapflaster oder einer Trachealkanüle, allgemeiner einer Hautoberfläche eines Benutzers, der insbesondere solche Befestigungsmittel für die erfindungsgemäße Vorrichtung trägt, verstanden. Beispielsweise bedeutet ein Verschluss distal eines Filtermaterials, dass bei der Führung der Ventilplatte aus einer Offenstellung in eine Geschlossenstellung das Filtermaterial vollständig auf der dem Körper des Benutzers zugewandten Seite von der Ventilplatte, dass heißt proximal, angeordnet ist.

Wird im Rahmen der Erfindung der Begriff "im Wesentlichen" verwendet, so gibt dieses einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

In einer Ausführungsform umfasst das Gehäuse zumindest eine Filteraufnahme. In einer Ausgestaltung ist das Gehäuse zumindest teilweise zylindrisch ausgestaltet. Das Gehäuse umfasst in einer Ausgestaltung eine Gehäusewandung. Bevorzugt umfasst die Filteraufnahme die Gehäusewandung. Weiter bevorzugt ist die Gehäusewandung im Wesentlichen zylindrisch ausgestaltet. Bevorzugt umfasst die Gehäusewandung eine distale und eine proximale Stirnfläche, die weiter bevorzugt eben ausgestaltet sind. Bevorzugt ist der proximalen Seite der Gehäusewandung ein Respirationsschutz zugeordnet. In einer weiteren Ausgestaltung ist der distalen Seite der Gehäusewandung, insbesondere der distalen Stirnfläche eine Krempe zugeordnet. Die Krempe erstreckt sich bevorzugt vollständig umlaufend radial von der Gehäusewandung weg. Insbesondere ist eine distale Oberfläche der Krempe auf der gleichen Ebene wie die distale Stirnfläche der Gehäusewandung angeordnet. In einer weiteren Ausführungsform umfasst das Gehäuse einen Rahmen, der bevorzugt distal der Filteraufnahme angeordnet ist. Er kann jedoch auch zumindest teilweise auf gleicher Höhe angeordnet sein wie das Filtermaterial, und dessen Oberseite liegt im Wesentlichen in einer Ebene mit einer distalen Oberfläche des Filtermaterials. In einer weiteren bevorzugten Ausführungsform ist vorgesehen, dass zwischen Rahmen und Filteraufnahme Streben angeordnet sind, die den Rahmen von der Filteraufnahme beabstanden. Bevorzugt ist der Rahmen ringförmig ausgestaltet. Weiter bevorzugt weist der Rahmen einen Außendurchmesser auf, der etwa einem Außendurchmesser der Filteraufnahme entspricht. In einer weiteren Ausführungsform weist der Rahmen einen Innendurchmesser auf, der größer ist als ein Außendurchmesser der Ventilplatte. Bevorzugt wird die Ventilplatte nicht von dem Rahmen gehalten oder in ihrer insbesondere transversalen Bewegung begrenzt. Vorteil des Rahmens ist, dass beispielsweise ein versehentliches Betätigen der Ventilplatte, insbesondere ein versehentliches Verschließen des Wärme- und Feuchtigkeitsaustauschers, beispielsweise durch Kleidung, vermieden werden kann. Insbesondere sind die Streben derart angeordnet, dass zwischen den Streben eine Ventilation stattfinden kann. In einer Ausgestaltung ist vorgesehen, dass zumindest drei, weiter bevorzugt etwa drei bis etwa fünf Streben vorgesehen sind.

Die Filteraufnahme ist vorzugsweise im Wesentlichen zylindrisch ausgestaltet, wobei diese zumindest eine distale Öffnung, und weiter bevorzugt zumindest eine proximale Öffnung aufweist. Weiter bevorzugt ist ein Mantel der Filteraufnahme gasdicht ausgestaltet und weist insbesondere keine Öffnungen auf. In einer weiter bevorzugten Ausgestaltung weist die Filteraufnahme einen Respirationsschutz auf. Der Respirationsschutz ist vorzugsweise der proximalen Öffnung der Filteraufnahme zugeordnet. In einer Ausgestaltung umfasst der Respirationsschutz ein Gitter, ein Sieb, eine oder mehrere Lamellen, eine Lochplatte und/oder ein oder mehrere Streben, insbesondere ein Strebenkreuz. In einer Ausgestaltung ist der Respirationsschutz sternförmig ausgestaltet. Der Respirationsschutz ist insbesondere derart ausgestaltet, dass dieser das Filtermaterial zurück hält, so dass dieses nicht versehentlich vom Benutzer des Wärme- und Feuchtigkeitsaustauschers eingeatmet wird.

Im Sinne der vorliegenden Erfindung ist unter einer Geschlossenstellung des Wärme- und Feuchtigkeitsaustauschers eine Position der Ventilplatte zu verstehen, bei der die an der Ventilplatte angeordnete vorzugsweise umlaufende Lippe in die Nut des Ventilsitzes eingreift und insbesondere auf dem Nutgrund aufliegt. Weiterhin zeichnet sich die Geschlossenstellung dadurch aus, dass eine distale Öffnung des Gehäuses, insbesondere eine distale Öffnung der Filteraufnahme, im Wesentlichen luftdicht verschlossen ist.

Erfindungsgemäß umfasst das Gehäuse den Ventilsitz. Der Ventilsitz umschließt die distale Öffnung des Gehäuses und ist bevorzugt derart ausgestaltet, dass mittels der Ventilplatte die distale Öffnung des Gehäuses, insbesondere der Filteraufnahme, im Wesentlichen luftdicht verschließbar ist. Die Nut zur Aufnahme der Lippe ist bevorzugt vollständig umlaufend ausgestaltet. Weiter bevorzugt öffnet die Nut in Richtung distal. In einer weiteren Ausgestaltung ist vorgesehen, dass die Nut zur Aufnahme der Lippe bevorzugt eine längliche, bevorzugt winklige Vertiefung im Material des Ventilsitzes ist. Vorzugsweise ist die Nut beziehungsweise der Ventilsitz derart ausgestaltet, dass bei Verschluss des Wärme- und Feuchtigkeitsaustauschers die Lippe in die Nut eingreift. In einer Ausgestaltung liegt die Ventilplatte, insbesondere ein Teil der proximalen Oberfläche der Ventilplatte, bei Verschluss des Wärme- und Feuchtigkeitsaustauschers auf dem Ventilsitz, insbesondere auf der distalen Stirnseite der Gehäusewandung und/oder der distalen Oberfläche der Krempe, auf. In einer weiteren Ausgestaltung greift die Lippe, insbesondere in einer Geschlossenstellung, in die Nut ein, so dass die proximale Oberfläche der Ventilplatte nicht auf dem Ventilsitz aufliegt, insbesondere liegt die Ventilplatte nicht auf einer distalen Stirnseite der Filteraufnahme oder einer distalen Oberfläche der Krempe auf, da die Lippe von der Ventilplatte weiter nach proximal ragt als die Nut tief ist. Das bedeutet, der Wärme- und Feuchtigkeitsaustauscher ist durch Eingreifen der Lippe in die Nut verschlossen, bevor ein Teil der proximalen Oberfläche der Ventilplatte auf dem Ventilsitz, insbesondere auf einer distalen Stirnseite der Filteraufnahme oder einer der distalen Oberfläche der Krempe, aufliegt. In einer Ausführungsform beträgt die Tiefe der Nut etwa 0,1 mm bis etwa 0,5 mm, bevorzugt etwa 0,2 mm bis 0,3 mm, weiter bevorzugt etwa 0,2 mm. In einer weiteren Ausgestaltung ist vorgesehen, dass die Nut des Ventilsitzes eine Tiefe aufweist, die etwa 10 % bis etwa 90 %, weiter bevorzugt etwa 80 % bis etwa 90 %, weiter bevorzugt etwa 85 % bis etwa 90 %, der proximalen Erstreckung der Lippe aufweist. In einer weiteren Ausgestaltung ist vorgesehen, dass die Nut eine Breite aufweist, die das etwa 1,1-Fache bis etwa 2-Fache, bevorzugt das etwa 1,5-Fache der Breite der Lippe aufweist. Im Sinne der Erfindung ist unter der Tiefe der Nut eine Erstreckung der Nut von einer distalen Stirnfläche des Gehäuses, insbesondere der Filteraufnahme, und/oder der distalen Oberfläche der Krempe bis zu einem Nutgrund zu verstehen.

Der Nutgrund ist insbesondere gekennzeichnet durch die im Wesentlichen tiefste Stelle der Nut, die in einer Ausführungsform eben ist. In einer weiteren Ausgestaltung ist vorgesehen, dass der Nutgrund den Abschnitt der Nut umfasst, auf dem die Lippe zur Auflage kommt.

Der Vorteil der Abdichtung mittels einer Lippe, die in eine Nut eingreift, ist, dass der Benutzer bei Verschluss des Wärme- und Feuchtigkeitsaustauschers eine taktile Rückmeldung bekommt, wenn die Lippe in die Nut eingreift beziehungsweise wenn das Ventil verschlossen ist. Weiterhin wird die Dichtwirkung durch die labyrinthartige Dichtung von Nut und Lippe erhöht. Ein weiterer Vorteil der Dichtung in der vorgeschlagenen Abdichtung des Wärme- und Feuchtigkeitsaustauschers ist, dass beim Verschließen die Ventilplatte durch Eingriff der Lippe in die Nut zentriert wird und somit ein sicherer Sitz der Ventilplatte auf dem Ventilsitz gewährleistet ist.

In einer weiteren Ausgestaltung ist vorgesehen, dass die Nut einen U-förmigen, V-förmigen, rechteckigen oder trapezförmigen Querschnitt aufweist. Insbesondere umfasst eine insbesondere trapezförmige Nut einen sich nach distal erweiternden Querschnitt auf. Bevorzugt ist das die Nut umgebende Material des Ventilsitzes im Wesentlichen eben. Insbesondere ist die Nut eine längliche, insbesondere winklige Vertiefung in einer im Wesentlichen ebenen Materialoberfläche.

In einer weiteren Ausgestaltung ist vorgesehen, dass der Ventilsitz eine das Gehäuse umlaufende Krempe aufweist. Insbesondere umrandet die Krempe die Filteraufnahme. In einer weiteren Ausgestaltung ist vorgesehen, dass die Krempe eine radial nach außen kragende Materialanhäufung ist, welche bevorzugt eine im Wesentlichen ebene distale Oberfläche aufweist. Weiter bevorzugt ist vorgesehen, dass die Krempe am distalen Ende der Filteraufnahme angeordnet ist. Weiter bevorzugt ist vorgesehen, dass eine distale Oberfläche der Krempe unmittelbar an eine distale Stirnseite der Filteraufnahme angrenzt und bevorzugt mit dieser eine im Wesentlichen ebene Oberfläche bildet, in der die Nut angeordnet ist. Weiter bevorzugt umfasst der Ventilsitz die distale Oberfläche der Krempe und die distale Stirnseite der Filteraufnahme. Die Nut ist vorzugsweise in der distalen Oberfläche der Krempe und/oder der distalen Stirnseite der Filteraufnahme angeordnet. Mittels der Krempe ist in einer Ausführungsform eine Auflagefläche der Ventilplatte größer als wenn die Ventilplatte nur auf der Stirnseite des Filtersitzes aufliegt. Weiterhin vorteilhaft erlaubt die Krempe eine Anpassung des Radius der Nut und/oder der Breite der Nut an einen Radius der Ventilplatte und/oder einer Breite der Lippe. Ein weiterer Vorteil der Krempe ist eine stabile Anordnung der Streben, die insbesondere den Rahmen des Gehäuses halten. Die Streben sind vorzugsweise auf der Krempe und/oder der distalen Stirnseite der Filteraufnahme angeordnet. Innen und außen sind insbesondere ausgehend von der Gehäusewandung zu verstehen.

In einer weiteren Ausgestaltung ist vorgesehen, dass das Gehäuse eine Zapfenaufnahme aufweist. Insbesondere ist die Zapfenaufnahme in der Filteraufnahme, weiter bevorzugt zentral in der Filteraufnahme, angeordnet. Besonders bevorzugt ist vorgesehen, dass die Zapfenaufnahme distal bis zur Höhe eines Randes des Gehäuses, insbesondere bis zur Höhe der distalen Stirnfläche der Filteraufnahme beziehungsweise der Gehäusewandung, ragt. In einer weiteren Ausgestaltung weist die Zapfenaufnahme eine Höhe von proximal nach distal auf, die kleiner ist als die Höhe der Wandung des Gehäuses, insbesondere der Filteraufnahme. Bevorzugt ist die Zapfenaufnahme auf dem Respirationsschutz angeordnet. Insbesondere ist die Zapfenaufnahme distal des Respirationsschutzes im Gehäuse angeordnet. In einer weiteren Ausgestaltung ist Zapfenaufnahme mit dem Respirationsschutz materialverbunden. In einer weiteren Ausgestaltung erstreckten sich zumindest Teile des Respirationsschutzes zumindest teilweise ausgehend von der Zapfenaufnahme radial in Richtung Gehäusewandung. Bevorzugt ist vorgesehen, dass der Respirationsschutz ein Strebenkreuz bildet, in dessen Zentrum die Zapfenaufnahme angeordnet ist. Das Strebenkreuz kann dreiarmig oder vierarmig ausgestaltet sein. In einer weiteren Ausgestaltung weist das Strebenkreuz beliebig viele Arme, insbesondere mehr als vier Arme, auf.

In einer weiteren Ausgestaltung ist vorgesehen, dass die Zapfenaufnahme eine Rastaufnahme aufweist. Insbesondere ist die Zapfenaufnahme zumindest teilweise als zumindest eine Rastaufnahme, weiter bevorzugt als zumindest ein federndes Funktionselement einer Schnappverbindung, ausgestaltet. Weiter bevorzugt ist vorgesehen, dass die Zapfenaufnahme etwa zwei bis etwa fünf, weiter bevorzugt etwa drei bis etwa vier Rastaufnahmen aufweist. Die Rastaufnahmen sind vorzugsweise elastisch, insbesondere im Wesentlichen federelastisch, ausgebildet. Vorzugsweise wird ein federnder Teil der Schnappverbindung durch die Rastaufnahme gebildet.

Allgemein wird unter einer Schnappverbindung eine Verbindung von zumindest zwei Funktionselementen verstanden, die gefügt werden. Dabei verformt sich zumindest ein Funktionselement federnd beziehungsweise elastisch und verhakt sich anschließend lösbar oder unlösbar mit dem zumindest zweiten Funktionselement. Beispielsweise umfasst die Zapfenaufnahme etwa zwei bis etwa fünf, bevorzugt etwa vier Rastaufnahmen, die als federndes Funktionselement ausgestaltet sind und insbesondere einen Rücksprung aufweisen. Weiter bevorzugt weist der Zapfen einen vorzugsweise umlaufenden Rastvorsprung auf. Beim Fügen von Zapfen in die Zapfenaufnahme werden die Rastaufnahmen von dem Zapfen elastisch verbogen, bis der Rastvorsprung hinter den Rücksprung greift und die Rastaufnahmen einschnappen. Bevorzugt bilden Zapfenaufnahme und Zapfen eine formschlüssige Verbindung.

In einer weiteren Ausführungsform ist vorgesehen, dass die Ventilplatte einen Zapfen umfasst. In einer bevorzugten Ausgestaltung ist vorgesehen, dass die Ventilplatte einen Zapfen umfasst, der in einer Zapfenaufnahme des Gehäuses insbesondere transversal führbar ist. Insbesondere ist in einer Ausführungsform vorgesehen, dass der Zapfen ein im Wesentlichen starres Element der Schnappverbindung bildet. Weiter bevorzugt ist die Zapfenaufnahme derart ausgestaltet, dass die Ventilplatte transversal von proximal nach lateral und zurück führbar ist. Insbesondere ist die Ventilplatte relativ zum Gehäuse etwa 6 mm bis etwa 9 mm, bevorzugt etwa 6 mm bis etwa 7 mm, transversal bewegbar. Weiter bevorzugt ist vorgesehen, dass mittels einer Erstreckung der Zapfenaufnahme nach distal die Bewegung der Ventilplatte nach proximal begrenzt ist. Insbesondere ist die Bewegung der Ventilplatte mittels Aufliegen der Ventilplatte auf der Zapfenaufnahme nach proximal begrenzt. In einer weiteren Ausführungsform ist vorgesehen, dass mittels der formschlüssigen Verbindung von Rastvorsprung und Rastaufnahme beziehungsweise Zapfen und Zapfenaufnahme, insbesondere mittels der Schnappverbindung, eine Bewegung in Richtung distal begrenzt ist. Vorzugsweise ist in einer Ausgestaltung vorgesehen, dass eine Bewegungsbegrenzung der Ventilplatte in Richtung proximal mittels Eingreifen der Lippe in die Nut beziehungsweise Aufliegen der Lippe auf einen Nutgrund begrenzt ist. In einer weiteren Ausführungsform ist vorgesehen, dass eine Bewegung nach distal der Ventilplatte derart begrenzt ist, dass diese sich im Wesentlichen auf Höhe des Rahmens befindet. In einer weiteren Ausgestaltung ist vorgesehen, dass die Zapfenaufnahme zentral in der Filteraufnahme des Gehäuses angeordnet ist.

In einer weiteren vorteilhaften Ausgestaltung ist vorgesehen, dass der Wärme- und Feuchtigkeitsaustauscher ein Federelement umfasst, das der Ventilplatte zugeordnet ist. Bevorzugt ist das Federelement derart angeordnet, dass dieses eine Rückstellkraft auf die Ventilplatte bewirkt, wenn die Ventilplatte mittels einer Verschlusskraft in Richtung proximal bewegt wird. Insbesondere bewirkt das Federelement, dass die Ventilplatte in eine Offenstellung zurückgeführt wird, nachdem die Verschlusskraft nicht mehr vorhanden ist.

In einer weiteren Ausgestaltung ist vorgesehen, dass das Federelement einen zentralen Ring zur Aufnahme des Zapfens umfasst. Vorzugsweise ist mittels des zentralen Rings sichergestellt, dass die Rückstellkraft, die durch das Federelement auf die Ventilplatte ausgeübt wird, im Wesentlichen gleichmäßig und weiter bevorzugt im Mittel zentral auf die Ventilplatte wirkt, insbesondere um ein Verkanten der Ventilplatte beim Zurückführen in die Offenstellung zu vermeiden.

In einer weiter bevorzugten Ausgestaltung umfasst das Federelement insbesondere ausgehend von dem zentralen Ring etwa zwei bis etwa acht, weiter bevorzugt etwa zwei bis etwa vier, weiter bevorzugt genau vier Federstreben. Die Federstreben sind vorzugsweise als Blattfedern ausgestaltet. In einer weiteren Ausgestaltung ist vorgesehen, dass zumindest zwei Federstreben eine T-förmige Ausgestaltung aufweisen. Insbesondere weisen die T-förmigen Federstreben einen sich radial erstreckenden Stamm und ein im Wesentlichen rechtwinklig zum Stamm angeordnetes Querstück auf. Die als Blattfedern ausgestalteten Federstreben erstrecken sich im Wesentlichen nur radial und weisen keine Querstücke auf. Weiter bevorzugt ist vorgesehen, dass zumindest zwei Federstreben, insbesondere blattfederförmige Federstreben, eine Krümmung aufweisen, die im eingebauten Zustand nach proximal, oder bevorzugt nach distal, ragen. Insbesondere ist in einer Ausgestaltung vorgesehen, dass die Querstücke der T-förmig ausgestalteten Federstreben eine Biegung nach proximal oder, besonders bevorzugt, nach distal aufweisen. In einer weiteren Ausgestaltung ist vorgesehen, dass die Stämme der T-förmigen Federstreben nach distal gekrümmt sind. In einer weiteren Ausgestaltung ist vorgesehen, dass die Stämme der T-förmigen Federstreben nach proximal gekrümmt sind. In einer weiteren Ausgestaltung ist vorgesehen, dass die Stämme der T-förmigen Federstreben nicht gekrümmt sind. In einer Ausgestaltung weisen die Querstücke eine Biegung nach proximal auf. In einer weiteren Ausgestaltung weisen die Querstücke eine Biegung nach distal auf. Besonders bevorzugt ist vorgesehen, dass zwei T-förmige Federstreben rechtwinklig zu zwei als Blattfedern ausgestalteten Federstreben angeordnet sind. Weiter bevorzugt sind die Federstreben auf dem Umfang des zentralen Ringes im Wechsel blattfederförmig und T-förmig verteilt. In einer weiteren Ausgestaltung ist vorgesehen, dass das Federelement vier blattfederförmige Federstreben, die insbesondere nach distal gekrümmt sind, aufweist. In einer weiteren Ausgestaltung ist keine T-förmige Federstrebe vorgesehen, sondern ausschließlich blattfederförmige Federstreben. Im Sinne der Erfindung entspricht ein Querstück der T-förmigen Federstrebe bildlich dem Deckstrich des Buchstabens T. Weiterhin umfasst der Stamm der T-förmigen Federstrebe bevorzugt den Abschnitt vom Ring bis zum Querstück.

In einer weiteren bevorzugten Ausgestaltung ist vorgesehen, dass in der Filteraufnahme ein Filtermaterial angeordnet ist. Das Filtermaterial kann insbesondere mindestens einen Schaumstoff umfassen. Besonders bevorzugt ist vorgesehen, dass das Filtermaterial im Wesentlichen bündig mit der distalen Stirnseite der Filteraufnahme endet. In einer weiteren Ausführungsform ist vorgesehen, dass das Filtermaterial im Wesentlichen bündig mit der distalen Erstreckung der Zapfenaufnahme endet. Besonders bevorzugt ist vorgesehen, dass das Filtermaterial ein Schaumstoffzylinder ist, der insbesondere eine zentrale Öffnung zur Aufnahme der Zapfenaufnahme aufweist. In einer weiteren bevorzugten Ausgestaltung ist vorgesehen, dass der Radius des Filtermaterials etwa dem Innenradius der Filteraufnahme entspricht. In einer weiteren Ausführungsform ist vorgesehen, dass das Filtermaterial in einer Presspassung in die Filteraufnahme eingeführt ist.

In einer Ausgestaltung liegt das Federelement auf dem Filtermaterial auf. In einer weiteren Ausführungsform ist vorgesehen, dass das Federelement auf der Zapfenaufnahme aufliegt. Insbesondere liegt der zentrale Ring des Federelementes auf der Zapfenaufnahme auf. In dieser Ausgestaltung stützt sich das Federelement nicht oder nur unwesentlich auf dem Filter auf und ein indirektes Auspressen beim Verschließen des Wärme- und Feuchtigkeitsaustauschers wird verhindert. Vorzugsweise erstrecken sich die Federstreben ausgehend etwa von der Zapfenaufnahme und/oder von dem Filtermaterial in Richtung der Ventilplatte. In einer Ausführungsform liegen die T-förmigen Federstreben auf dem Filtermaterial auf und sorgen vorzugsweise für eine Stabilisierung des Federelementes.

In einer weiteren Ausgestaltung ist vorgesehen, dass das Filtermaterial über einen distalen Rand der Filteraufnahme ragt. In einer weiteren Ausführungsform ist vorgesehen, dass das Filtermaterial bis zur Ventilplatte ragt, wenn die Ventilplatte beziehungsweise der Wärme- und Feuchtigkeitsaustauscher in einer Offenstellung ist. In einer weiteren Ausführungsform ist das Filtermaterial derart beschaffen, dass eine Rückstellkraft auf die Ventilplatte mittels des Filtermaterials ausgeübt wird. Weiter bevorzugt ist in einer Ausgestaltung vorgesehen, dass sowohl das Filterelement als auch ein Federelement eine Rückstellkraft auf die Ventilplatte ausübt. In einer weiteren Ausgestaltung ist vorgesehen, dass kein Federelement vorgesehen ist. In einer Ausführungsform ist vorgesehen, dass das Filtermaterial derart ausgestaltet ist, dass dieses insbesondere allein, das hießt, ohne dass ein sonstiges Federelement vorhanden ist, eine Rückstellkraft auf die Ventilplatte ausüben kann, also eine federnde Funktion übernimmt. In einer weiteren Ausführungsform ist vorgesehen, dass die Ventilplatte auf dem Filtermaterial befestigt, insbesondere aufgeklebt oder verschweißt, ist. Diese Ausgestaltung ist insbesondere dann vorgesehen, wenn die Zapfenaufnahme in Verbindung mit dem Zapfen die Ventilplatte nicht hält beziehungsweise die Bewegung in Richtung distal nicht begrenzt. Weiterhin kann die Ventilplatte auf dem Filtermaterial aufgeklebt oder angeschweißt sein, wenn beispielsweise der Wärme- und Feuchtigkeitsaustauscher keine Zapfenaufnahme und/oder keinen Zapfen aufweist. Nachteilig an der Verklebung ist allerdings, dass durch das Aufbringen von Kleber dieser in das Filtermaterial eindringt und eine Aufnahmekapazität insbesondere für Flüssigkeit des Filtermaterials einschränkt. Eine bevorzugte Ausgestaltung des Wärme- und Feuchtigkeitsaustauschers ohne Federelement weist die Zapfenaufnahme und den in dieser angeordneten Zapfen der Ventilplatte auf. Diese Ausgestaltung sichert eine definierte transversale Führung beim Schließen und Öffnen des Wärme- und Feuchtigkeitsaustauschers. Zudem wirkt die Zapfenverbindung als Verlierschutz der Ventilplatte, ohne die Filterkapazität des Filtermaterials zu beeinträchtigen.

Vorzugsweise umfasst die Ventilplatte ein Material ausgewählt aus einer Gruppe umfassend Polyamid, Polycarbonat, Polyoxymethylene, auch Polyacetalharz genannt, vorzugsweise Delrin® 500P NC010 - POM der Firma DuPont Engineering Polymers, Acrylnitril-Butadien-Styrol-Copolymere (ABS) und/oder eine Mischung aus zumindest zwei der genannten Materialien. Insbesondere ist vorgesehen, dass die Oberflächenbeschaffenheit des Materials, insbesondere der distalen Oberfläche der Ventilplatte, eine feinteilige und/oder homogene Morphologie aufweist. Weiterhin umfasst die Oberfläche der Ventilplatte einen geringen Glanzgrad.

Vorzugsweise umfasst das Gehäuse ein Material ausgewählt aus einer Gruppe umfassend Polyamid, Polycarbonat, Polypropylen, vorzugsweise ein statistisches Polypropylen-Copolymer, weiter bevorzugt Bormed™ RF825MO der Firma Borealis AG, Acrylnitril-Butadien-Styrol-Copolymere (ABS) und/oder eine Mischung aus zumindest zwei der genannten Materialien. Insbesondere ist vorgesehen, dass die Oberflächenbeschaffenheit des Materials des Gehäuses, insbesondere der Oberfläche des Ringes, der Streben und/oder der Filteraufnahme, eine feinteilige und/oder homogene Morphologie aufweist. Weiterhin umfasst die Oberfläche des Gehäuses einen geringen Glanzgrad.

Das Federelement weist vorzugsweise ein elastisches Material auf. Insbesondere weist das Material des Federelementes zumindest teilweise linear-elastische Eigenschaften auf. Insbesondere kann dieses gebildet sein aus Polystyrol, Polycarbonat, thermoplastischem Kunststoff, insbesondere Polyacton, insbesondere einem halbkristallinen Thermoplasten mit einer alternierende Struktur aus Kohlenmonoxid und Olefin, weiter bevorzugt Poketone™ M 930A der Firma Hyosung und/oder aus einem metallischen Werkstoff. Auch andere Materialien, die eine hinreichende Elastizität zur Verfügung stellen, sind möglich. Besonders bevorzugt weist das Federelement eine gleichmäßige Stärke auf. Die Stärke kann in einer weiteren Ausgestaltung insbesondere im Bereich eines Überganges, beispielsweise eines Übergangs zwischen dem Ring und den Federstreben, verringert sein. Das Filtermaterial umfasst in einer Ausgestaltung ein Material auf ausgewählt aus einer Gruppe umfassend mindestens einen offenporigen Schaumstoff, ein Tissuematerial und/oder ein Papier.

In einer ersten beispielhaften Ausführungsform umfasst der Wärme- und Feuchtigkeitsaustauscher die Ventilplatte, ein Federelement und ein Gehäuse und bevorzugt ein Filtermaterial, das in dem Gehäuse anordenbar ist. Die Ventilplatte weist distal eine Oberseite und proximal eine Unterseite auf. Die Ventilplatte wird umrandet von einer an der Unterseite angeordneten Lippe, die in Richtung des Gehäuses beziehungsweise nach proximal ragt. Weiterhin umfasst die Ventilplatte einen Zapfen, der ebenfalls im zusammengebauten Zustand nach proximal ragt. Der Zapfen ist derart gestaltet, dass dieser von der Zapfenaufnahme des Gehäuses aufnehmbar ist. Die Zapfenaufnahme umfasst bevorzugt eine Rastaufnahme, die bevorzugt aus vier Segmenten besteht. Ein Rücksprung verhindert in Verbindung mit dem Rastvorsprung des Zapfens ein Herausgleiten beziehungsweise Herausfallen der Ventilplatte aus der Zapfenaufnahme. Die distale Erstreckung des Ventilsitzes beziehungsweise der Filteraufnahme ist bevorzugt etwa auf der Höhe der maximalen distalen Erstreckung der Zapfenaufnahme angeordnet.

Bevorzugt liegt das Federelement im Wesentlichen auf der Zapfenaufnahme auf, kann aber auch so gestaltet sein, dass dieses auf dem Filtermaterial aufliegt. Das Federelement weist insbesondere einen zentralen Ring auf, der zu Montagezwecken einen Schlitz aufweist, um den Ring um den Zapfen zu montieren. Der Zapfen der Ventilplatte ragt durch den Ring in die Zapfenaufnahme. Das Federelement weist in der gezeigten Ausführungsform zwei Federstreben auf, die in Form einer Blattfeder in Richtung distal gebogen sind. Die Federstreben sind an dem Ring gegenüberliegend angeordnet. Rechtwinklig zu den Federstreben sind zwei weitere T-förmige Federstreben an dem Ring angeordnet. Querbalken, die insbesondere den Deckstrich des Buchstabens T bilden, der T-förmigen Federstreben sind bevorzugt ebenfalls nach distal gebogen. Bei Betätigung der Ventilplatte und Bewegung derselben nach proximal werden zumindest die Blattfederstreben in Richtung proximal verbogen, wobei diese eine Rückstellkraft auf die Ventilplatte ausüben. Eine Verschlusskraft in Richtung proximal kann die Ventilplatte verschließen, wobei die Lippe in die Nut eingreift. Mittels des durch Schließen der Ventilplatte verbogenen Federelements wird bevorzugt eine Rückstellkraft auf die Ventilplatte ausgeübt, welche die Ventilplatte wieder in die Offenstellung überführt, wenn die Verschlusskraft wegfällt oder nachlässt.

In einer zweiten beispielhaften Ausführungsform umfasst der Wärme- und Feuchtigkeitsaustauscher die Ventilplatte und ein Gehäuse und bevorzugt ein Filtermaterial, das in dem Gehäuse anordenbar ist. Die Ventilplatte umfasst eine distale Oberseite und eine proximale Unterseite. Die Ventilplatte wird umrandet von einer Lippe, die in Richtung des Gehäuses beziehungsweise nach proximal ragt. Das Filtermaterial erstreckt sich nach distal über eine distale Stirnseite der Filteraufnahme hinaus bis zur Ventilplatte in der Offenstellung. Weiterhin umfasst die Ventilplatte in dieser Ausgestaltung keinen Zapfen und weiter bevorzugt das Gehäuse keine Zapfenaufnahme. Die Ventilplatte kann beispielsweise mit dem Filtermaterial klebend oder verschweißt verbunden sein. In einer weiteren Ausgestaltung kann die Ventilplatte derart ausgestaltet sein, dass diese von dem Gehäuse, insbesondere von dem Rahmen, gehalten ist. Beispielsweise umfasst die Ventilplatte Anschlagelemente, die proximal unter den Rahmen greifen.

In einer dritten beispielhaften Ausgestaltung umfasst der Wärme- und Feuchtigkeitsaustauscher die Ventilplatte und ein Gehäuse und bevorzugt ein Filtermaterial, das in dem Gehäuse anordenbar ist. Die Ventilplatte weist distal eine Oberseite und proximal eine Unterseite auf. Die Ventilplatte wird umrandet von einer an der Unterseite angeordneten Lippe, die in Richtung des Gehäuses beziehungsweise nach proximal ragt. Weiterhin umfasst die Ventilplatte einen Zapfen, der ebenfalls im zusammengebauten Zustand nach proximal ragt. Der Zapfen ist derart gestaltet, dass dieser von der Zapfenaufnahme des Gehäuses aufnehmbar ist. Die Zapfenaufnahme umfasst bevorzugt eine Rastaufnahme, die bevorzugt aus vier Segmenten besteht. Ein Rücksprung verhindert in Verbindung mit dem Rastvorsprung des Zapfens ein Herausgleiten beziehungsweise Herausfallen der Ventilplatte aus der Zapfenaufnahme. Die distale Erstreckung des Ventilsitzes beziehungsweise der Filteraufnahme ist bevorzugt etwa auf der Höhe der maximalen distalen Erstreckung der Zapfenaufnahme angeordnet. Das Filtermaterial erstreckt sich nach distal über eine distale Stirnseite der Filteraufnahme hinaus bis zur Ventilplatte in der Offenstellung. Ein Federelement ist nicht vorgesehen.

In allen beispielhaften Ausgestaltungen kann die Oberfläche der Oberseite der Ventilplatte eine Vertiefung nach proximal aufweisen. Insbesondere ist die Vertiefung dazu ausgestaltet, dass diese komfortabel eine menschliche Fingerkuppe zum Teil aufnehmen und auch führen kann. Insbesondere ist eine sich nach distal erstreckende konzentrische Wölbung im Wesentlichen auf einer Höhe mit einer maximalen distalen Erstreckung der distalen Seite des Rahmens angeordnet. Die sich nach distal erstreckende Wölbung hat im Querschnitt einen Radius von bevorzugt etwa 14,1 mm. Die sich nach proximal erstreckende Wölbung hat im Querschnitt einen Radius von bevorzugt etwa 40 mm. Bevorzugt sind die Wölbung und die Vertiefung konzentrisch zueinander angeordnet. Bevorzugt ist die Ventilplatte in der Offenstellung im Wesentlichen auf einer Höhe des Rahmens angeordnet. Weiter bevorzugt ist die proximale Oberfläche der Ventilplatte im Wesentlichen auf der Höhe einer proximalen Stirnseite des Rahmens angeordnet, um vorteilhafter Weise einen möglichst großen Luftdurchtritt zu ermöglichen.

In allen beispielhaften Ausgestaltungen umfasst das Gehäuse einen Ventilsitz, welcher eine Nut umfasst. Bevorzugt ist die Nut bevorzugt derart gestaltet, dass die Lippe der Ventilplatte zumindest teilweise in diese aufgenommenen werden kann. Des Weiteren ist der Ventilsitz derart geformt, dass dieser die Krempe aufweist, die radial nach außen weisend um das Gehäuse umläuft und insbesondere an eine distale Stirnseite der Filteraufnahme angrenzt. Das Gehäuse umfasst bevorzugt des Weiteren Streben, die von der Filteraufnahme beziehungsweise der Krempe nach distal ragen und den Rahmen halten, der ein versehentliches Betätigen der Ventilplatte verhindern kann. Der Rahmen hat insbesondere eine Höhe von proximal nach distal, die einer maximalen Höhe der Ventilplatte entspricht. Der Rahmen hat in einer weiteren Ausgestaltung eine Höhe von proximal nach distal, die einer minimalen Höhe der Ventilplatte entspricht. Ein Luftstrom in und aus dem Wärme- und Feuchtigkeitsaustauscher erfolgt im zusammengebauten Zustand im Wesentlichen durch Ventilationsöffnungen, die zwischen den Streben, dem Rahmen und dem Ventilsitz angeordnet sind. Insbesondere umrahmen je zwei Streben, ein Abschnitt des Rahmens und ein Abschnitt des Ventilsitzes eine Ventilationsöffnung. Das proximale Ende des Gehäuses umfasst einen Respirationsschutz, der verhindert, dass das Filtermaterial von dem Benutzer des Wärme- und Feuchtigkeitsaustauschers in den Körper gelangt und insbesondere von diesem eingeatmet wird. Des Weiteren trägt der Respirationsschutz in einer Ausgestaltung zentral in der Filteraufnahme die Zapfenaufnahme, sofern eine Zapfenaufnahme vorgesehen ist.

In der zweiten und dritten beispielhaften Ausgestaltung ist vorgesehen, dass der Wärme- und Feuchtigkeitsaustauscher kein Federelement aufweist. Vielmehr ist vorgesehen, dass mittels des Filtermaterials eine Rückstellkraft auf die Ventilplatte ausübbar ist. In dieser Ausgestaltung wird beim Einbringen einer Verschlusskraft durch Verformen des Filtermaterials eine Rückstellkraft erzeugt. Das Filtermaterial ist über den distalen Rand der Filteraufnahme erstreckt und reicht bei unbetätigtem Wärme- und Feuchtigkeitsaustauscher an die Ventilplatte in der Offenstellung. In einer Ausgestaltung ist die Ventilplatte gegen Verlieren durch den Zapfen und die Zapfenaufnahme, wie oben beschrieben, gesichert. In einer weiteren Ausgestaltung, insbesondere wenn der Wärme- und Feuchtigkeitsaustauscher keinen Zapfen und/oder keine Zapfenaufnahme aufweist, ist die Ventilplatte mit dem Filtermaterial verklebt. In einer weiteren Ausgestaltung ist die Ventilplatte mit dem Filtermaterial verschweißt. In einer weiteren Ausgestaltung umfasst die Ventilplatte Vorsprünge, die radial nach außen vorstehen und in die Ventilationsöffnungen eingreifen, damit die Ventilplatte in ihrer Bewegung nach distal begrenzt wird. Auch anderweitige Sicherungsmechanismen gegen Verlieren können verwendet werden.

Bevorzugt ist in einer beispielhaften Ausgestaltung vorgesehen, dass die umlaufende Lippe in der Offenstellung des Wärme- und Feuchtigkeitsaustauschers distal von der Nut beabstandet angeordnet ist. Die Nut ist in der radial nach außen kragenden Krempe des Ventilsitzes angeordnet und weist eine Tiefe auf, die etwa 0,1 mm bis etwa 0,3 mm, bevorzugt 0,2 mm beträgt. Die Lippe wiederum ragt etwa 0,5 mm bis etwa 0,9 mm, bevorzugt etwa 0,7 mm ausgehend von der Unterseite der Ventilplatte nach proximal.

Weiterhin wird ein Verfahren zum Verschließen eines Wärme- und Feuchtigkeitsaustauschers wie oben beschrieben vorgeschlagen, wobei eine Ventilplatte nach proximal bewegt wird, wobei eine Lippe der Ventilplatte in eine Nut eines Gehäuses eingreift. Durch dieses Verfahren kann ein luftdichter Verschluss der distalen Öffnung des Gehäuses beziehungsweise der Filteraufnahme erzeugt werden. Vorzugsweise greift die Lippe der Ventilplatte derart in die Nut ein, dass die Ventilplatte relativ zum Gehäuse zentriert wird. Weiterhin vorteilhaft ist vorgesehen, dass mittels des Eingriffs der Lippe in die Nut eine taktile Rückmeldung erfolgt, die dem Benutzer anzeigt, dass der Wärme- und Feuchtigkeitsaustauscher in einer Geschlossenstellung ist.

In einer weiteren Ausgestaltung ist vorgesehen, dass die Ventilplatte gegen eine Rückstellkraft, die von einem Federelement ausgeübt wird, nach proximal bewegt wird. Vorteilhafterweise ist die Rückstellkraft, beziehungsweise eine Federkonstante des Federelementes, derart eingestellt, dass diese die Ventilplatte in eine Offenstellung zurück bewegt, wenn die Verschlusskraft nach proximal wegfällt oder geringer ist als die Rückstellkraft.

In einer weiteren Ausführungsform ist vorgesehen, dass die Ventilplatte gegen eine Rückstellkraft, die von einem Filtermaterial ausgeübt wird, nach proximal bewegt wird. Vorteilhafterweise ist das Filtermaterial derart beschaffen, dass dieses die Rückstellkraft derart ausübt, dass diese die Ventilplatte in eine Offenstellung zurück bewegt, wenn die Verschlusskraft nach proximal wegfällt oder geringer ist als die Rückstellkraft. Weiterhin vorteilhaft wird die Ventilplatte bei der transversalen Bewegung in die Offenstellung und die Geschlossenstellung durch die Zapfenverbindung geführt.

Weiterhin wird die Verwendung eines Wärme- und Feuchtigkeitsaustauschers zur Umleitung eines Luftstromes vorgeschlagen. Weiterhin wird auch die Verwendung eines Wärme- und Feuchtigkeitsaustauschers zum Blockieren eines Luftstromes vorgeschlagen. Der Wärme- und Feuchtigkeitsaustauscher kann insbesondere einer Person, die tracheotomiert oder laryngektomiert wurde, ermöglichen, ausgeatmete Luft durch ein Shunt oder die Larynx zu leiten. In einer weiteren Ausgestaltung wird der oben beschriebene Wärme- und Feuchtigkeitsaustauscher zur taktilen Rückmeldung beim Überführen einer Ventilplatte in die Geschlossenstellung verwendet.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zur weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
- Fig. 1: einen Wärme- und Feuchtigkeitsaustauscher in einer Explosionsansicht;
- Fig. 2: eine Schnittansicht des zusammengesetzten Wärme- und Feuchtigkeitsaustauschers aus Fig. 1 in einer Offenstellung;
- Fig. 3: eine Schnittansicht des zusammengesetzten Wärme- und Feuchtigkeitsaustauschers aus Fig. 1 in einer Geschlossenstellung; und
- Fig. 4: eine alternative Ausgestaltung des Wärme- Feuchtigkeitsaustauschers.

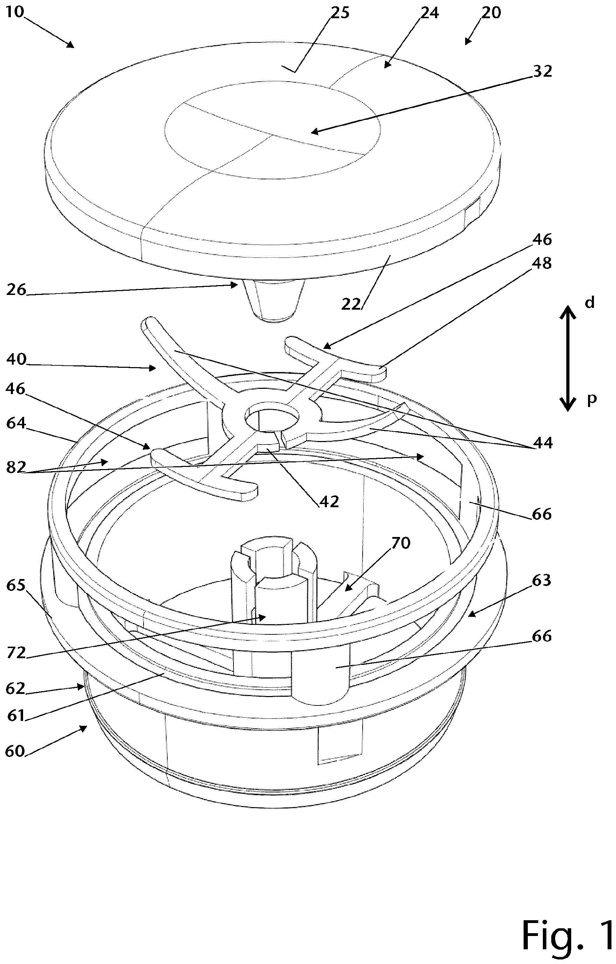

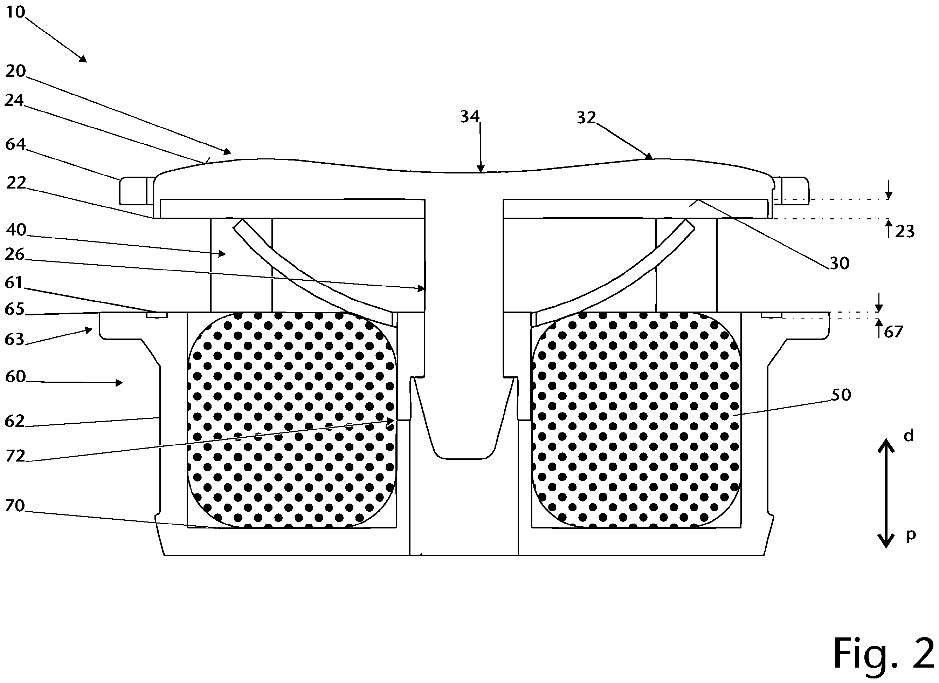

Fig. 1 zeigt einen Wärme- und Feuchtigkeitsaustauscher 10 in einer Explosionsansicht. Der Wärme- und Feuchtigkeitsaustauscher 10 umfasst die Ventilplatte 20, ein Federelement 40 und ein Gehäuse 60. Ein Filtermaterial 50, das in dem Gehäuse 60 anordenbar ist, ist der Übersichtlichkeit halber in Fig. 1 nicht dargestellt. Die Ventilplatte 20 umfasst eine Oberseite 24 und eine in Fig. 2 dargestellte Unterseite 30. Die Ventilplatte 20 wird umrandet von einer Lippe 22, die in Richtung des Gehäuses 60 beziehungsweise nach proximal p ragt. Weiterhin umfasst die Ventilplatte 20 einen Zapfen 26, der ebenfalls im zusammengebauten Zustand nach proximal p ragt. Der Zapfen 26 ist derart gestaltet, dass dieser von der Zapfenaufnahme 72 des Gehäuses 60 aufnehmbar ist. Die Oberfläche 25 der distalen Oberseite 24 der Ventilplatte 20 weist eine Wölbung 32 nach proximal auf. Insbesondere ist die Wölbung 32 dazu ausgestaltet, dass diese komfortabel eine menschliche Fingerkuppe zum Teil aufnehmen kann.

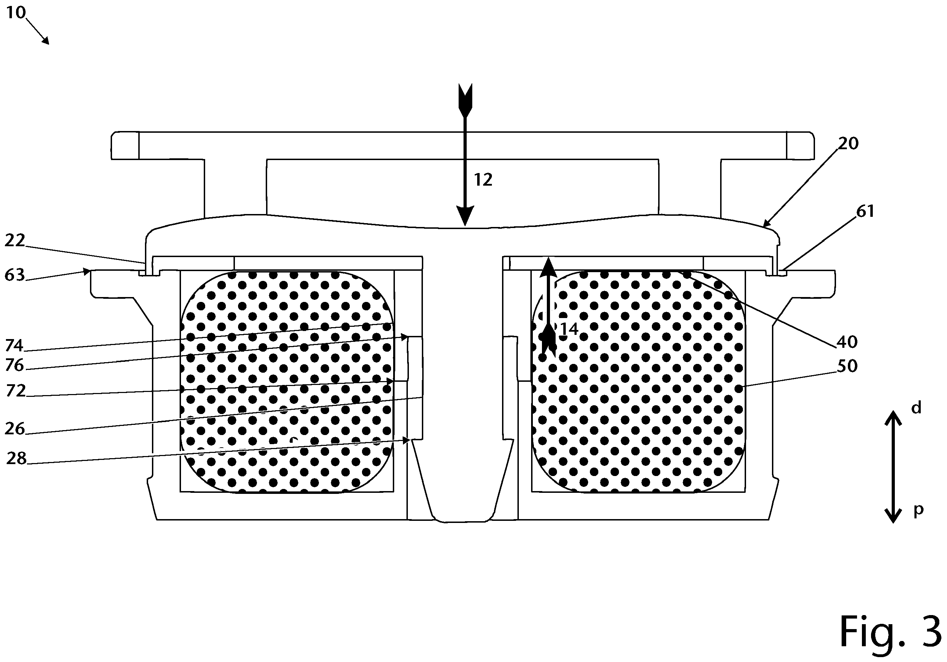

Das Gehäuse 60 weist einen Ventilsitz 63 auf, welcher eine Nut 61 umfasst. Die Nut 61 ist derart gestaltet, dass die Lippe 22 der Ventilplatte 20 zumindest teilweise in diese aufgenommenen werden kann. Des Weiteren ist der Ventilsitz 63 derart geformt, dass dieser eine Krempe 65 aufweist, die radial nach außen weisend um das Gehäuse 60 umläuft. Insbesondere ist der Fig. 1 zu entnehmen, dass der Ventilsitz 63 distal d an einer Filteraufnahme 62 angeordnet ist. Das Gehäuse 60 umfasst des Weiteren Streben 66, die von der Filteraufnahme 62 nach distal d ragen und einen Rahmen 64 halten, der ein versehentliches Betätigen der Ventilplatte 20 verhindern kann. Ein Luftstrom in und aus dem Wärme- und Feuchtigkeitsaustauscher 10 erfolgt im zusammengebauten Zustand im Wesentlichen durch die Ventilationsöffnungen 82, die zwischen den Streben 66, dem Rahmen 64 und dem Ventilsitz 63 angeordnet sind. Das proximale Ende des Gehäuses 60 umfasst einen Respirationsschutz 70, der verhindert, dass das in Fig. 1 nicht gezeigte Filtermaterial 50 von dem Benutzer des Wärme- und Feuchtigkeitsaustauschers 10 in den Körper gelangt. Des Weiteren trägt der Respirationsschutz 70 zentral in der Filteraufnahme 62 die Zapfenaufnahme 72. In dem Wärme- und Feuchtigkeitsaustauscher 10 ist des Weiteren ein Federelement 40 angeordnet, das im zusammengebauten Zustand des Wärme- und Feuchtigkeitsaustauschers 10 im Wesentlichen auf der Zapfenaufnahme 72 aufliegt. Das Federelement 40 weist einen zentralen Ring 42 auf, der zu Montagezwecken einen nicht weiter bezeichneten Schlitz aufweist. Der Zapfen 26 der Ventilplatte 20 ragt im zusammengebauten Zustand durch den Ring 42 in die Zapfenaufnahme 72. Das Federelement 40 weist in der gezeigten Ausführungsform zwei Federstreben 44 auf, die in Form einer Blattfeder in Richtung distal gebogen sind. Die Federstreben 44 sind an dem Ring 42 gegenüberliegend angeordnet. Rechtwinklig zu den Federstreben 44 sind T-förmige Federstreben 46 an dem Ring 42 angeordnet. Wie Fig. 1 weiterhin zu entnehmen ist, sind die Querbalken 48 (die den Deckstrich des Buchstabens T bilden) der T-förmigen Federstreben 46 ebenfalls nach distal gebogen. Bei Betätigung der Ventilplatte 20 und Bewegung derselben nach proximal p werden zumindest die Federstreben 44 in Richtung proximal p verbogen, wobei diese eine Rückstellkraft 14, die in Fig. 3 angedeutet ist, auf die Ventilplatte 20 ausüben.

Fig. 2 zeigt eine Schnittansicht des Wärme- und Feuchtigkeitsaustauschers 10 in einer Offenstellung. Zu erkennen ist die Ventilplatte 20, deren Zapfen 26 in die Zapfenaufnahme 62 des Gehäuses 60 ragt. Weiterhin ist die umlaufende Lippe 22 zu erkennen, die distal d von der Nut 61 beabstandet angeordnet ist. Die Nut 61 ist in der radial nach außen kragenden Krempe 65 des Ventilsitzes 63 angeordnet und weist eine Tiefe 67 auf, die etwa 0,5 mm bis etwa 1,5 mm beträgt. Die Lippe 22 wiederum ragt etwa 0,5 mm bis etwa 1,5 mm ausgehend von der Unterseite 30 der Ventilplatte 20 nach proximal p.

Fig. 2 ist weiterhin zu entnehmen, dass der Rahmen 64 im Wesentlichen auf der Höhe der Ventilplatte 20 angeordnet ist. Insbesondere ist zu erkennen, dass eine sich nach distal d erstreckende konzentrische Wölbung 34 mit einer maximalen distalen Erstreckung auf der Höhe der distalen Seite des Rahmens 64 angeordnet ist. Ebenfalls ist die sich nach proximal p erstreckende, konzentrisch umlaufende Wölbung 32 der der Oberfläche 24 zu erkennen. Die sich nach distal erstreckende Wölbung 34 hat im Querschnitt einen Radius von etwa 14,1 mm. Die sich nach proximal erstreckende Wölbung 32 hat im Querschnitt einen Radius von etwa 40 mm. Der Fig. 2 ist weiterhin zu entnehmen, dass die distale Erstreckung des Ventilsitzes 63 beziehungsweise einer Filteraufnahme 62 etwa auf der Höhe der maximalen distalen Erstreckung der Zapfenaufnahme 72 ist. Ein Filtermaterial 50 ist in der Filteraufnahme 62 angeordnet und erstreckt sich vom Respirationsschutz 70 bis zur distalen Kante des Ventilsitzes 63.

Fig. 3 zeigt den Wärme- und Feuchtigkeitsaustauscher 10 in einer Geschlossenstellung. Eine Verschlusskraft 12 in Richtung proximal schließt die Ventilplatte 20, wobei die Lippe 22 in die Nut 61 eingreift. Mittels des durch Schließen der Ventilplatte 20 verbogenen Federelements 40 wird eine Rückstellkraft 14 auf die Ventilplatte 20 ausgeübt, welche die Ventilplatte 20 wieder in die Offenstellung überführt, wenn die Verschlusskraft 12 wegfällt oder nachlässt.

Des Weiteren ist der Fig. 3 zu entnehmen, dass die Zapfenaufnahme 72 eine Rastaufnahme 74 aufweist, die, wie in Fig. 1 zu sehen ist, aus vier Segmenten besteht. Ein Rücksprung 76 verhindert in Verbindung mit dem Rastvorsprung 26 des Zapfens 72 ein Herausgleiten beziehungsweise Herausfallen der Ventilplatte 20 aus der Zapfenaufnahme 72.

Durch Eingreifen der Lippe 22 in die Nut 61 des Ventilsitzes 63 wird die Ventilplatte zentriert. Durch die dadurch entstehende Reibung erfährt der Benutzer eine taktile Rückmeldung über das Schließen des Wärme- und Feuchtigkeitsaustauschers 10. Weiterhin wird durch die Verbindung von Lippe 22 und Nut 61 eine verbesserte Dichtigkeit des Wärme- und Feuchtigkeitsaustauschers 10 in der Geschlossenstellung erzielt. Das Filtermaterial 50 wird in dieser Ausgestaltung in der Geschlossenstellung nicht komprimiert.

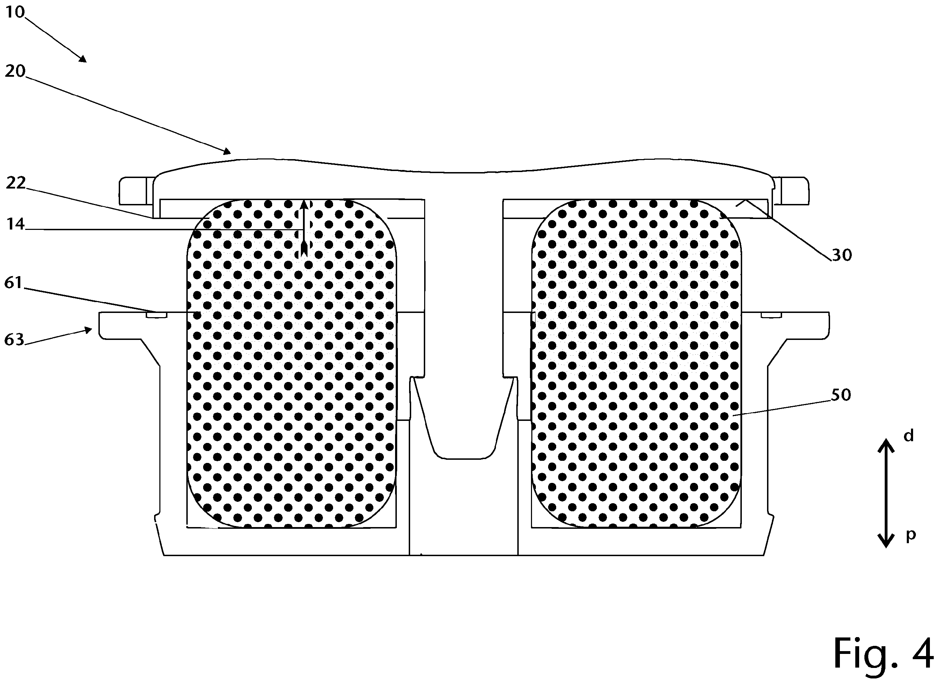

Fig. 4 zeigt eine alternative Ausgestaltung des Wärme- Feuchtigkeitsaustauschers 10 ohne ein Federelement. Eine Rückstellkraft 14, die in der nicht gezeigten Geschlossenstellung des Wärme- und Feuchtigkeitsaustauschers 10 die Ventilplatte 20 nach distal in die Offenstellung treibt, wird mittels des Filtermaterials 50 erzeugt. Das Filtermaterial 50 erstreckt sich ausgehend von dem Respirationsschutz 70 distal über den Ventilsitz 63 hinaus bis zur Unterseite 30 der Ventilplatte 20 in Offenstellung.

Ein weiterer Vorteil der Dichtung in der vorgeschlagenen Abdichtung des Wärme- und Feuchtigkeitsaustauschers 10 in allen vorgeschlagenen Ausführungsformen ist, dass beim Verschließen die Ventilplatte 20 durch Eingriff der Lippe 22 in die Nut 61 zentriert wird und somit ein sicherer Sitz der Ventilplatte 20 auf dem Ventilsitz 63 gewährleistet ist.

Des Weiteren ist eine nicht in den Figuren gezeigte Ausgestaltung vorgesehen, bei dem kein Zapfen 26 und/oder keine Zapfenaufnahme 72 vorgesehen ist. Die Rückstellkraft 14 auf die Ventilplatte 20 kann wie in Fig. 4 illustriert durch das Filtermaterial 50 ausgeübt werden. Weiterhin ist vorgesehen, dass die Ventilplatte 20 mittels Verkleben oder Verschweißen mit dem Filtermaterial 50 verbunden ist, damit die Ventilplatte 20 nicht verloren geht. In einer weiteren Ausgestaltung weist die Ventilplatte 20 in den Figuren nicht gezeigte Vorsprünge auf, die proximal unter den Rahmen 64 greifen, um die Ventilplatte 20 zu sichern.

Des Weiteren ist eine in den Figuren ebenfalls nicht gezeigte Ausgestaltung vorgesehen, bei der die Ventilplatte 20 eine im Wesentlichen ebene distale Oberfläche 25 aufweist. Auch andere Konturen der distalen Oberfläche, die in den Figuren nicht gezeigt sind, sind in verschiedenen Ausgestaltungen vorgesehen. Weiterhin ist in den Figuren eine Ausgestaltung des Respirationsschutzes 70 vorgesehen, bei der dieser als ein Gitter, ein Sieb, eine oder mehrere Lamellen, eine Lochplatte, eine Sternform und/oder als 3, 5 oder mehr Streben ausgestaltet ist. Insbesondere können die Streben des Respirationsschutzes gleichmäßig oder ungleichmäßig in Umfangsrichtung verteilt sein.

Mit dem vorgeschlagenen Wärme- und Feuchtigkeitsaustauscher wird dem tracheostomierten oder laryngektomierten Benutzer eine komfortable Möglichkeit gegeben, einen Sprechvorgang einzuleiten und beim Betätigen des Wärme- und Feuchtigkeitsaustauschers eine taktile Rückmeldung über den Verschluss zu erhalten. Weiterhin ist durch die Anordnung der Lippe in der Nut in der Geschlossenstellung ein sicherer Verschluss des Wärme- und Feuchtigkeitsaustauschers gewährleistet. Die in einer Ausgestaltung vorgeschlagene Wölbung der distalen Oberseite der Ventilplatte bietet zudem den Komfort, mit dem Finger angenehm zentral auf die Ventilplatte drücken zu können, um die Verschlusskraft optimal einzuleiten.

## Patentansprüche

1. Wärme- und Feuchtigkeitsaustauscher (10) für Tracheostomierte oder Laryngektomierte, zumindest umfassend eine Ventilplatte (20) und ein Gehäuse (60), wobei die Ventilplatte (20) eine radial umlaufende Lippe (22) umfasst und wobei das Gehäuse (60) einen eine distale Öffnung (78) des Gehäuses (60) umschließenden Ventilsitz (63) umfasst, wobei der Ventilsitz (63) eine Nut (61) zur Aufnahme der Lippe (22) umfasst, wobei die Ventilplatte (20) der distalen Öffnung (78) des Gehäuses (60) zugeordnet ist und wobei die Ventilplatte (20) in eine Geschlossenstellung überführbar ist, bei der die Lippe (22) in die Nut (61) eingreift.

2. Wärme- und Feuchtigkeitsaustauscher (10) nach Anspruch 1, **dadurch gekennzeichnet, dass** die Nut (61) des Ventilsitzes (63) eine Tiefe (67) aufweist, die etwa 10 % bis etwa 90 % der proximalen Erstreckung (23) der Lippe (22) aufweist.

3. Wärme- und Feuchtigkeitsaustauscher (10) nach einem oder mehreren der vorhergehenden Ansprüche, **dadurch gekennzeichnet, dass** die Nut (61) einen U-förmigen oder V-förmigen Querschnitt aufweist.

4. Wärme- und Feuchtigkeitsaustauscher (10) nach einem oder mehreren der vorhergehenden Ansprüche, **dadurch gekennzeichnet, dass** der Ventilsitz (63) eine das Gehäuse (60) umlaufende Krempe (65) aufweist, wobei die Krempe (65) die Nut (61) umfasst.

5. Wärme- und Feuchtigkeitsaustauscher (10) nach einem oder mehreren der vorhergehenden Ansprüche, **dadurch gekennzeichnet, dass** die Ventilplatte (20) einen Zapfen (26) umfasst, der in einer Zapfenaufnahme (72) des Gehäuses (60) führbar ist.

6. Wärme- und Feuchtigkeitsaustauscher (10) nach einem oder mehreren der vorhergehenden Ansprüche, **dadurch gekennzeichnet, dass** die Zapfenaufnahme (72) zentral in einer Filteraufnahme (62) des Gehäuses angeordnet ist.

7. Wärme- und Feuchtigkeitsaustauscher (10) nach einem oder mehreren der vorhergehenden Ansprüche, **dadurch gekennzeichnet, dass** dieser ein Federelement (40) umfasst, das der Ventilplatte (20) zugeordnet ist.

8. Wärme- und Feuchtigkeitsaustauscher (10) nach Anspruch 7, **dadurch gekennzeichnet, dass** das Federelement (40) einen zentralen Ring (42) zur Aufnahme des Zapfens (26) umfasst.

9. Verfahren zum Verschließen eines Wärme- und Feuchtigkeitsaustauschers (10) nach einem oder mehreren der vorhergehenden Ansprüche, **dadurch gekennzeichnet, dass** eine Ventilplatte (20) nach proximal bewegt wird, wobei eine Lippe (22) der Ventilplatte (20) in eine Nut (63) eines Gehäuses (60) eingreift.

10. Verfahren nach Anspruch 9, **dadurch gekennzeichnet, dass** die Ventilplatte (20) gegen eine Rückstellkraft (14), die von einem Federelement (40) ausgeübt wird, nach proximal bewegt wird.

## Claims

1. A heat and moisture exchanger (10) for tracheotomy or laryngectomy patients, comprising at least a valve plate (20) and a housing (60), wherein the valve plate (20) comprises a radial circumferential limp (22), and wherein the housing (60) comprises a valve seat (63) that encompasses a distal opening (78) of the housing (60), wherein the valve seat (63) comprises a groove (61) for receiving the lip (22), wherein the valve plate (20) is dedicated to the distal opening (78) of the housing (60), and wherein the valve plate (20) can be closed, such that the lip (22) engages in the groove (61).

2. The heat and moisture exchanger (10) according to claim 1, **characterized in that** the groove (61) in the valve seat (63) has a depth (67) that is approx. 10% to approx. 90% of the proximal extension (23) of the lip (22).

3. The heat and moisture exchanger (10) according to either or both of the preceding claims, **characterized in that** the groove (61) has a U-shaped or V-shaped cross section.

4. The heat and moisture exchanger (10) according to any one or more of the preceding claims, **characterized in that** the valve seat (63) has a rim (65) encircling the housing (60), wherein the rim (65) comprises the groove (61).

5. The heat and moisture exchanger (10) according to any one or more of the preceding claims, **characterized in that** the valve plate (20) comprises a pin (26) that can be inserted into a pin receiver (72) in the housing (60).

6. The heat and moisture exchanger (10) according to any one or more of the preceding claims, **characterized in that** the pin receiver (72) is located in the center of a filter receiver (62) in the housing.

7. The heat and moisture exchanger (10) according to any one or more of the preceding claims, **characterized in that** it comprises a spring element (40) dedicated to the valve plate (20).

8. The heat and moisture exchanger (10) according to claim 7, **characterized in that** the spring element (40) comprises a central ring (42) for receiving the pin (26).

9. A method for closing a heat and moisture exchanger (10) according to any one or more of the preceding claims, **characterized in that** a valve plate (20) is moved proximally, wherein a lip (22) on the valve plate (20) engages in a groove (63) in the housing (60).

10. The method according to claim 9, **characterized in that** the valve plate (20) is moved proximally, counter to a return force (14) exerted by a spring element (40).

## Revendications

1. Échangeur de chaleur et d'humidité (10) pour les trachéotomisés ou les laryngectomisés, comprenant au moins une plaque de valve (20) et un boîtier (60), ladite plaque de valve (20) comprenant une lèvre radialement circonférentielle (22) et ledit boîtier (60) comprenant un siège de valve (63) renfermant une ouverture distale (78) dudit boîtier (60), ledit siège de valve (63) comprenant une rainure (61) pour ladite réception de la lèvre (22), ladite plaque de valve (20) est associé à ladite ouverture distale (78) dudit boîtier (60) et ladite plaque de valve (20) est déplaçable dans une position fermée dans laquelle ladite lèvre (22) s'engage dans ladite rainure (61).

2. Échangeur de chaleur et d'humidité (10) selon la revendication 1, **caractérisé en ce que** ladite rainure (61) dudit siège de valve (63) a une profondeur (67) qui est d'environ 10% à environ 90% de l'étendue proximale (23) de ladite lèvre (22).

3. Échangeur de chaleur et d'humidité (10) selon une ou plusieurs des revendications précédentes, **caractérisé en ce que** ladite rainure (61) a une coupe transversale en forme de U ou en V.

4. Échangeur de chaleur et d'humidité (10) selon une ou plusieurs des revendications précédentes, **caractérisé en ce que** ledit siège de valve (63) a un bord (65) entourant ledit boîtier (60), ledit bord (65) comprend ladite rainure (61).

5. Échangeur de chaleur et d'humidité (10) selon une ou plusieurs des revendications précédentes, **caractérisé en ce que** ladite plaque de valve (20) comprend un tourillon (26) qui est guidable dans un logement à tourillon (72) dudit boîtier (60).

6. Échangeur de chaleur et d'humidité (10) selon une ou plusieurs des revendications précédentes, **caractérisé en ce que** ledit logement à tourillon (72) est disposé centralement dans un logement à filtre (62) dudit boîtier.

7. Échangeur de chaleur et d'humidité (10) selon une ou plusieurs des revendications précédentes, **caractérisé en ce qu'**il comprend un élément ressort (40) qui est associé à ladite plaque de valve (20).

8. Échangeur de chaleur et d'humidité (10) selon la revendication 7, **caractérisé en ce que** ledit élément ressort (40) comprend un anneau central (42) pour la réception dudit tourillon (26).

9. Procédé de fermeture d'un échangeur de chaleur et d'humidité (10) selon une ou plusieurs des revendications précédentes, **caractérisé en ce qu'**une plaque de valve (20) est déplacée proximalement, une lèvre (22) de ladite plaque de valve (20) s'engage dans une rainure (63) d'un boîtier (60).

10. Procédé selon la revendication 9, **caractérisé en ce que** la plaque de valve (20) est déplacée proximalement contre une force de rappel (14) qui est exercée par un élément ressort (40).
